(19) Europäisches Patentamt
European Patent Office
Office européen des brevets

(11) Veröffentlichungsnummer: **0 238 569**
**B1**

(12) # EUROPÄISCHE PATENTSCHRIFT

(45) Veröffentlichungstag der Patentschrift: **14.12.88**

(51) Int. Cl.⁴: **C 07 C 61/06**, C 07 C 61/08, C 07 C 51/00, C 07 C 1/28

(21) Anmeldenummer: **86905728.1**

(22) Anmeldetag: **24.09.86**

(86) Internationale Anmeldenummer: **PCT/DE 86/00396**

(87) Internationale Veröffentlichungsnummer: **WO 87/02034 (09.04.87 Gazette 87/08)**

(54) **VERFAHREN ZUR HERSTELLUNG VON CYCLOALKANCARBONSÄUREN.**

(30) Priorität: **26.09.85 DE 3534613**

(43) Veröffentlichungstag der Anmeldung: **30.09.87 Patentblatt 87/40**

(45) Bekanntmachung des Hinweises auf die Patenterteilung: **14.12.88 Patentblatt 88/50**

(84) Benannte Vertragsstaaten: **AT BE CH DE FR GB IT LI LU NL SE**

(56) Entgegenhaltungen: **US-A-3 450 782**

(73) Patentinhaber: **KYOWA HAKKO KOGYO KABUSHIKI KAISHA, 6-1, Ohte- machi 1-chome, Chiyoda- ku Tokyo 100 (JP)**

(72) Erfinder: **Mayr, Herbert, Prof.Dr., Meisenweg 1, D-2401 Gross- Grönau (DE)**
Erfinder: **Heilmann, Werner, Dr., Damaschkestrasse 13, D-2400 Lübeck 1 (DE)**
Erfinder: **Vorbrüggen, Helmut, Prof.Dr., Wilkestr. 7, D-1000 Berlin 27 (DE)**

(74) Vertreter: **Kinzebach, Werner, Dr., Patentanwälte Reitstötter, Kinzebach und Partner Sternwartstrasse 4 Postfach 86 06 49, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 238 569 B1

LIBER, STOCKHOLM 1988

**Beschreibung**

Die Erfindung betrifft das in den Patentansprüchen gekennzeichnete Verfahren.

Die Cycloalkancarbonsäuren der allgemeinen Formel I sind bekanntlich wertvolle Zwischenprodukte, die unter anderem zur Herstellung von Süßstoffen Verwendung finden (Europäische Patentanmeldung 0 129 654). Die Herstellung diser Verbindungen ist aber nach dem bekannten Stande der Technik sehr aufwendig.

Das erfindungsgemäße Verfahren ermöglicht es, diese Verbindungen auf wesentlich einfacherem Wege zu synthetisieren.

Die Ausgangssubstanzen für das erfindungsgemäße Verfahren können als Alkylgruppen $R_1$ bis $R_4$ beispielsweise Methylgruppen, Ethylgruppen, Propylgruppen, Isopropylgruppen oder Butylgruppen enthalten. Im Hinblick auf die gewerbliche Verwertbarkeit der Verfahrensprodukte sind solche Ausgangsverbindungen bevorzugt, deren Substituenten $R_1$ bis $R_4$ identisch sind. Besonders bevorzugte Ausgangsverbindungen sind solche, die als Substituenten $R_1$ bis $R_4$ Methylgruppen tragen. Als Substituenten X und X' können die Ausgangssubstanzen gleiche oder unterschiedliche Gruppen tragen. Diese Gruppen können polare Substituenten sein, die unter Ausbildung von Carbeniumionen abspaltbar sind. Solche Substituenten sind vorzugsweise Chloratome, Bromatome, Alkylsulfonyloxygruppen (wie die Methansulfonyloxygruppe oder die Trifluormethansulfonyloxygruppe) oder Arylsulfonyloxygruppen (wie die Benzolsulfonyloxygruppe, die p-Brombenzolsulfonyloxygruppe oder die p-Toluolsulfonyloxygruppe). Diese Ausgangsverbindungen können in einfacher Weise aus den entsprechenden Diolen durch Veresterung oder durch Austausch der Hydroxygruppen gegen Chlor oder Brom hergestellt werden. Bevorzugte Ausgangsverbindungen sind solche, die als Substituenten X und X' Methansulfonyloxygruppen oder insbesondere Chloratome oder Bromatome tragen.

In der ersten Stufe des erfindungsgemäßen Verfahrens werden die Verbindungen der allgemeinen Formel II unter an sich bekannten Bedingungen mit 1,1-Dichlorethylen umgesetzt (siehe beispielsweise: Angew. Chem. 78, 1966, 932 und 91, 1980, 169; Tetrahedron Letters 1968, 4979 und 1973, 1569; Chem. Ber. 100, 1967, 978; 103, 1970, 3851 und 106, 1973, 2513; J. Chem. Soc. Perk. Ed. I, 1973, 2559; Acta. Chem. Scand. 34b, 1980, 621 und Synth. Comm. 14, 1984, 113; J. Amer. Chem. Soc., 67, 1945, 1152; 68, 1946, 1650; 46, 1946, 1655 und 71, 1949, 698 sowie J. Org. Chem. 48, 1983, 1159). So kann man beispielsweise die Verbindungen der Formel II in 1,1-Dichlorethylen als Lösungsmittel unter Zusatz starker Lewis-Säuren (wie beispielsweise Eisen(III)-chlorid, Eisen(III)-bromid, Zinn(IV)-chlorid, Zinn(IV)-bromid, Zirkonium(IV)-chlorid, Bortrichlorid, Bortrifluorid, Zink(II)-chlorid, Gallium(III)-chlorid oder insbesondere Aluminium(III)-chlorid) bei einer Reaktionstemperatur von -50°C bis +100°C umsetzen und erhält die Verbindungen der Formel III. Andererseits ist es aber auch möglich, diesen Reaktionsschritt unter Verwendung anderer, unter den Reaktionsbedingungen inerter Lösungsmittel (beispielsweise chlorierter Kohlenwasserstoffe) durchzuführen.

Aus den so dargestellten Verbindungen der allgemeinen Formel III wird in einen zweiten Reaktionsschritt, der unter den Bedingungen durchgeführt werden kann, welche ebenfalls in den obengenannten Publikationen beschrieben sind, Chlorwasserstoff oder gegebenenfalls auch HX abspaltet. Zur Abspaltung verwendet man ökonomischerweise wäßrige Basen, wie Natronlauge oder Kalilauge. Oft ist es zur Erhöhung der Reaktionsgeschwindigkeit zweckmäßig, der Reaktionsmischung noch Phasentransferkatalysatoren (wie zum Beispiel Aliquat®) zuzusetzen. Dieser Reaktionschritt wird zweckmäßigerweise bei einer Reaktionstemperatur von 20°C bis 100°C durchgeführt.

Die Abspaltung von Chlorwasserstoff und gegebenfalls auch von HX' kann je nach Art des Substituenten X der Reaktionsbedingungen bereits während der ersten Stufe des erfindungsgemäßen Verfahrens erfolgen; dies ist beispielsweise immer der Fall, wenn X eine freie Hydoxygruppe ist.

In der dritten Stufe des erfindungsgemäßen Verfahrens werden die Verbindungen der allgemeinen Formel IV mittels Säuren cyclisiert und hydrolysiert. Für diesen Reaktionsschritt eignen sich als Säuren Mineralsäuren wie zum Beispiel Phosphorsäure, Polyphosphorsäure, Chlorwasserstoff und insbesondere Schwefelsäure, organische Sulfonsäure wie Methansulfonsäure oder p-Toluolsulfonsäure oder stark saure Carbonsäuren wie Trifluoressigsäure oder insbesondere auch Ameisensäure. Andererseits eignen sich beispielweise auch stark saure Ionenaustauscher wie Amberlite® IR 120 zur Durchführung dieser Reaktionsstufe. Dieser Reaktionsschritt kann ohne Verwendung zusätzlicher Lösungsmittel durchgeführt werden, dies insbesondere dann, wenn man als Säuren Phospohorsäure, Polyphosphorsäure, Schwefelsäure oder Ameisensäure verwendet. Anderseits ist es aber auch möglich, diese Reaktion in Gegenwart anderer Lösungsmittel wie zum Beispiel Eisessig durchzuführen. Die Reaktionstemperatur, bei der man diesen Reaktionsschritt durchführt ist von den Reaktionsbedingungen - insbesondere von der verwendeten Säure - abhängig. Sie beträgt in der Regel etwa -20°C bis 120°C.

Durch die Wahl geeigneter Reaktionsbedingungen ist es möglich zwei oder alle drei Stufen des erfindungsgemäßen Verfahrens in einer Eintopfreaktion durchzuführen.

Die nachfolgenden Ausführungsbeispiele dienen zur näheren Erläuterung des erfindungsgemäßen Verfahrens.

**Beispiel 1**

a) Zu einer auf 0°C gekühlten Suspension von 54,9 g 2,5-Dichlor-2,5-dimethyl-hexan in 135 ml 1,1-Dichlorethylen werden innerhalb von 15 Minuten 3,75 g wasserfreies Aluminiumchlorid gegeben. Man rührt die Reaktionsmischung nach 70 Minuten lang und zersetzt sie durch Zugabe von 30 g zerstoßenem Eis. Die organische Phase wird abgetrennt, mit 2 prozentiger Salzäure gewaschen, über Calziumchlorid getrocknet, im Vakuum fraktioniert und man erhält neben unumgesetzten Ausgangsmaterial das 1,1,1,6-Tetrachlor-3,3,6-trimethyl-heptan vom Siedepunkt 67°C bei 0,4 bar.

b) 1,68 g 1,1,1,6-Tetrachlor-3,3,6-trimethyl-heptan werden in 4 ml Dichlormethan mit 10 ml 40 %-iger wäßriger Natronlauge und 0,52 g Aliquat® 336 versetzt und 17 Stunden lang unter Rückfluß erhitzt. Dann läßt man die Reaktionsmischung erkalten, versetzt sie mit 20 ml Dichlormethan, trennt die organische Phase ab, wäscht sie mit Wasser und trocknet sie über Calziumchlorid. Dann engt man die Lösung mit Vakuum ein und erhält das 1,6-Dichlor-3,3,6-trimethyl-1-heptin als öliges Rohprodukt.

c) Das so erhaltene 1,6-Dichlor-3,3,6-trimethyl-1-heptin-Rohprodukt wird innerhalb von 15 Minuten bei 0°C unter starkem Rühren zu 10 ml konz. Schwefelsäure getropft. Man rührt dann noch 14 Stunden lang bei 0°C, gießt die Mischung auf 20 g Eis und extrahiert zweimal mit je 20 ml Diethylether. Die organische Phase wird abgetrennt und zweimal mit je 20 ml 10 %-iger wässriger Natriumcarbonat-lösung extrahiert. Man säuert die wässrige Phase mit konz. Salzsäure an und erhält die 2,2,5,5-Tetramethyl-cyclopentancarbonsäure als NMRspektroskopisch reine Substanz von Schmelzpunkt 127° - 129°C.

**Beispiel 2**

a) 25,2 g 1,1,1,6-Tetrachlor-3,3,6-trimethylheptan werden mit 0,60 g Aliquat (Tricaprylmethylammoniumchlorid) und 50 ml 40 gew.-%-iger wäßriger Natronlauge unter starkem Rühren auf 80°C erwärmt. Dann läßt man die Mischung erkalten, verdünnt sie mit 60 ml Dichlormethan, trennt die organische Phase ab, wäscht sie mit Wasser, trocknet sie mit Calziumchlorid und zieht das Lösungsmittel ab.

b) 8,69 g des gemäß Beispiel 2a hergestellten 1,6-Dichlor-3,3,6-trimethyl-1-heptin-Rohprodukts werden innerhalb von 30 Minuten in 80 ml siedende wasserfreie Ameisensäure getropft und dann 16 Stunden lang unter Rückfluß erhitzt. Dann läßt man die Mischung erkalten, setzt 140 ml Wasser zu und extrahiert viermal mit je 30 ml Diethylether. Die vereinigten organischen Phasen werden mit Wasser gewaschen und anschließend fünfmal mit je 20 ml 10 gew.-%-iger wäßriger Natriumcarbonatlösung ausgeschüttelt. Man säuert die wäßrige Phase mit konz. Salzsäure an und erhält die 2,2,5,5-Tetramethyl-cyclopentancarbonsäure als NMRspektroskopisch reine Substanz vom Schmelzpunkt 127 - 129°C.

**Beispiel 3**

a) Zu einer gut gerührten Suspension von 54,9 g 2,5-Dichlor-2,5-dimethylhexan in 135 ml 1,1-Dichlorethylen werden bei 0°C unter Rühren innerhalb von 45 Minuten portionsweise 3,75 g Aluminiumchlorid gegeben. Dann rührt man die Reaktionsmischung noch weitere 100 Minuten lang bei 0°C und versetzt sie mit 10 ml Wasser und 20 g wasserfreiem Kalziumchlorid. Dann filtriert man und zieht das überschüssige 1,1-Dichlorethylen im Vakuum ab. Man erhält 88,4 g eines zählen Öls aus dem durch Feststoffdestillation 17,5 g 2,5-Dichlor-2,5-dimethylhexan zurückgewonnen werden. Der Rückstand wird destilliert und man erhält 34,3 g 1,1,1,6-Tetrachlor-3.3,6-trimethylheptan als schwach gelbes Öl vom Siedepunkt 79 - 80°C bei 0,5 m bar.

b) 25,2 g 1,1,1,6-Tetrachlor-3,3,6-trimethylheptan werden mit 0,60 g Tricaprylmethylamoniumchlorid (Aliquat®336) und 50 ml 40 %-iger wässriger Natronlauge unter starkem Rühren 22 Stunden lang auf 80° C erhitzt. Dann läßt man die Mischung erkalten, versetzt mit 60 ml Dichlormethan, trennt die organische Phase ab und trocknet sie über Kalziumchlorid. Dann wird das Lösungsmittel im Vakuum abgezogen und der Rückstand destilliert. Man erhält so 14,7 g 1,6-Dichlor-3,3,6-trimethyl-1-heptin als gelbes Öl von Siedepunkt 39 - 43°C bei 1 m bar.

c) Zu 80 ml 90 %-iger Ameisensäure werden unter starkem Rühren 8,29 g 1,6-Dichlor-3,3,6-trimethyl-1-heptin getropft und 6 Stunden lang unter Rückfluß erhitzt. Man läßt die Mischung erkalten versetzt sie 300 ml Wasser und extrahiert sie zweimal mit je 50 ml Petrolether. Die vereinigten organische Phasen werden fünfmal mit je 30 ml 10 %-iger wässriger Natriumcarbonatlösung extrahiert. Die wässrige Phase wird mit konzentrierter Salzsäure angesäurt, das ausgefallene Produkt aus Petrolether umkristallisiert und man erhält 4,63 g 2,2,5,5-Tetramethyl-cyclopentancarbonsäure vom Schmelzpunkt 128 - 129 °C.

**Patentansprüche**

1. Verfahren zur Herstellung van Cycloalkancarbonsäuren der allgemeinen Formel I

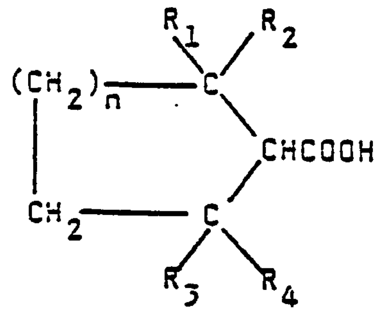

(I),

worin
n die Ziffer 1 oder 2 bedeutet,
$R_1$ und $R_3$ Alkylgruppen mit 1 bis 4 Kohlenstoffatomen darstellen und
$R_2$ und $R_4$ Wasserstoffatome oder Alkylgruppen mit 1 bis 4 Kohlenstoffatomen symbolisieren, dadurch gekennzeichnet, daß man eine Verbindung der allgemeinen Formel II

$X\text{-}CR_1R_2\text{-}(CH_2)_n\text{-}CH_2\text{-}CR_3R_4\text{-}X'$ (II),

worin
n, $R_1$, $R_2$, $R_3$ und $R_4$ die obengenannte Bedeutung besitzen und
X und X' eine Hydroxygruppe, ein Chloratom, ein Bromatom, ein Alkylsulfonyloxyrest oder ein Arylsulfonyloxyrest darstellen
mit 1,1-Dichlorethylen umsetzt
aus den so erhaltenen Verbindungen der allgemeinen Formel III

$X\text{-}CR_1R_2\text{-}(CH_2)_n\text{-}CH_2\text{-}CR_3R_4\text{-}CH_2\text{-}CCl_2X$ (III),

worin
n, X, X', $R_1$, $R_2$, $R_3$ und $R_4$ die obengenannte Bedeutung besitzen Chlorwasserstoff abspaltet und die so dargestellten Verbindungen der allgemeinen Formel IV

$X\text{-}CR_1R_2\text{-}(CH_2)_n\text{-}CH_2\text{-}CR_3R_4\text{-}CY{=}CZX'$ (IV),

worin
n, X, X', $R_1$, $R_2$, $R_3$ und $R_4$ die obengenannte Bedeutung besitzen und
Y und Z gemeinsam eine Kohlenstoff-Kohlenstoffbindung darstellen oder ein Wasserstoffatom und ein Chloratom bedeuten
mittels Säuren cyclisiert und hydrolysiert.

2. Verfahren zur Herstellung von Cycloalkancarbonsäuren der allgemeinen Formel I gemäß Patentanspruch 1, dadurch gekennzeichnet, daß man die Umsetzung mit 1,1-Dichlorethylen in Gegenwart von Lewis-Säuren als Katalysatoren durchführt.

**Claims**

1. Process for preparing cycloalkanecarboxylic acids of general formula I

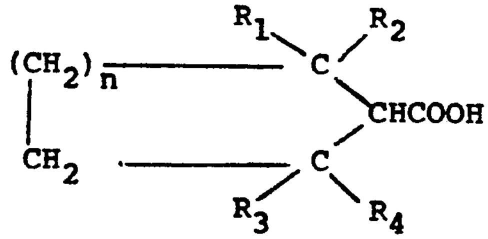

(I)

wherein
n represents the number 1 or 2,

$R^1$ and $R^3$ represent alkyl groups with one to four carbon atoms and
$R^2$ and $R^4$ represent hydrogen atoms or alkyl groups with one to four carbon atoms,
characterized in that a compound of general formula II

$X\text{-}CR_1R_2\text{-}(CH_2)_n\text{-}CH_2\text{-}CR_3R_4\text{-}X'$ (II)

wherein

n, $R_1$, $R^2$, $R^3$ and $R^4$ are defined as hereinbefore and X and X' represent a hydroxy group, a chlorine atom, a bromine atom, an alkylsulfonyloxy group or an arylsulfonyloxy group,

is reacted with 1,1-dichlorethylene,

hydrogen chloride is cleaved from the resulting compounds of general formula III

$X\text{-}CR_1R_2\text{-}(CH_2)_n\text{-}CH_2\text{-}CR_3R_4\text{-}CH_2\text{-}CH_2CCl_2X$ (III)

wherein

n, X, X', $R_1$, $R_2$ $R_3$ and $R_4$ are defined as hereinbefore and the resulting compounds of general formula IV

$X\text{-}CR_1R_2\text{-}(CH_2)_n\text{-}CH_2\text{-}CR_3R_4\text{-}CY{=}CZX'$ (IV)

wherein

n, X, X', $R_1$, $R_2$, $R_3$ and $R_4$ are defined as hereinbefore and

Y and Z together represent a carbon-carbon bond or a hydrogen atom and a chlorine atom are cyclised by means of acids and hydrolysed.

2. Process for preparing cycloalkancarboxylic acids of general formula I as claimed in claim 1, characterised in that the reaction is carried out with 1,1-dichlorethylene in the presence of Lewis acids as catalysts.

**Revendications**

1. Procédé de préparation d'acides cycloalcanecarboxyliques de formule générale I

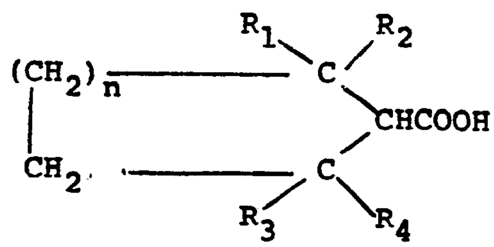

(I)

où

n représente le nombre 1 ou 2,

R1 et R3 représentent des groupes alcoyle en C1 à C4 et

R2 et R4 représentent des atomes d'hydrogène ou des groupes alcoyle en C1 à C4,

caractérisé en ce qu'on fait réagir un composé de formule générale II

$X\text{-}CR_1R_2\text{-}(CH_2)_n\text{-}CH_2\text{-}CR_3R_4\text{-}X'$ (II)

où

n, R1, R2, R3 et R4 ont la signification donnée ci-dessus et

X et X' représentent un groupe hydroxy, un atome de chlore, un atome de brome, un reste alcoylsulfonyloxy ou un reste arylsulfonyloxy,

avec le 1,1-dichloréthylène,

qu'on soumet les composé ainsi obtenus, de formule générale III

$X\text{-}CR_1R_2\text{-}(CH_2)_n\text{-}CH_2\text{-}CR_3R_4\text{-}CH_2\text{-}CCl_2X$ (III),

où

n, X, X', R1, R2, R3 et R4 ont la signification donnée ci-dessus, à une réaction d'élimination d'acide chlorhydrique, pour obtenir les composés de formule générale IV

$X\text{-}CR_1R_2\text{-}(CH_2)_n\text{-}CH_2\text{-}CR_3R_4\text{-}CY{=}CZX'$ (IV),

où

n, X, X', R1, R2, R3 et R4 ont la signification donnée ci-dessus et

Y et Z représentent ensemble une liaison carbone-carbone ou un atome d'hydrogène et un atome de chlore, et que l'on cyclise et hydrolyse à l'aide d'acides les composés obtenus.

2. Procédé de préparation d'acides cycloalcanecarboxyliques de formule générale I selon la revendication 1, caractérisé en ce qu'on conduit la réaction avec le 1,1-dichloréthylène en présence d'acides de Lewis comme catalyseurs.